# EUROPEAN PATENT SPECIFICATION

(11) **EP 1 567 156 B1**
(45) Date of publication and mention of the grant of the patent: **26.08.2009**
(21) Application number: 03772460.6
(22) Date of filing: 19.11.2003
(51) Int. Cl.: A61K 31/44

(54) **HYDROXYPYRIDINONES FOR THE LOCAL TREATMENT OF SKIN MICROCIRCULATORY DISORDERS**
HYDROXYPYRIDINONE ZUR LOKALEN BEHANDLUNG VON HAUTMIKROZIRULATIONSSTÖRUNGEN
HYDROXYPYRIDINONES POUR LE TRAITEMENT LOCAL DE TROUBLES MICROCIRCULATOIRES DE LA PEAU

(30) Priority: 19.11.2002 IT MI20022447
(43) Date of publication of application: 31.08.2005
(73) Proprietor: RELIVIA SRL, 20122 Milano (IT)
(72) Inventor: GHISALBERTI, Carlo, CEP-04542-80 Sao Paulo (BR)
(74) Representative: Gislon, Gabriele
(86) International application number: PCT/IB2003/005222
(87) International publication number: WO 2004/045609

(56) References cited:
- WO-A-01/17497
- WO-A-98/13043
- WO-A-03/084538

## Description

### FIELD OF THE INVENTION

The present invention relates to the use of specific hydroxypyridones for the preparation of external composition for the topical treatment of skin microcirculatory disorders (SMD).

### BACKGROUND OF THE INVENTION

The dermatology science has classified a number of skin disorders linked to the cuteneous microcirculatory disarray.

Inflammatory leaky capillaries and/or the accidental blood extravasation cause the intradermal deposition of hemosiderin. These featuers are common in rosacea, chronic pigmented purpuras (Shamberg's and related diseases) and other SMD such as lichen aureus and actinic purpura. Similar SMD effects are prompted by external factors, exemplary as side-effect of systemic drugs (e.g. minocycline, ciprofloxacin) and oncologic drugs; the traumatic events such as trauma and hemorrhage, e.g. following sclerotherapy or lipoplasty intervention.

Cutaneous vasculitis is a SMD with a inflammatory component affecting the skin vessels, including capillaries, venules, arterioles and lymphatics.

Rosacea is the most common SMD, which primarily interests the convexities on face, often characterized by remissions and exacerbations, thereby producing flushing, erythema, telangiectasia, edema, papulopustules, ocular lesions and rhinophyma. The vessel leakage by flushing results in blotchy red areas in rosacea is further aggravated by the sun exposure, and may degenerate into papulopustular, phymatous and granulomatous forms. Sufferers are disproportionately of skin type I and II, wherein in USA the affected people are 3.85% of population, with similar prevalence on the world-wide fair-skinned populations.

The hemosiderin extravasation from primary vessel fragility or secondary to acute and chronic vessel impairments actually induces a pro-inflammatory condition onto the cutis, and provokes a discomforting aesthetic burden in the sufferers.

SMD sufferers are treated with a variety of medicaments including corticosteroids (e.g. hydrocortisone, clobetasol, betamethasone), antibiotics and anthistamines. The latter produce a symptomatic relief of the pruritus caused by the release of histamine in inflammatory reactions, may exacerbate angle-closure glaucoma and hyperthyroidism, whilst the prolonged use of corticosteroids may produce local and systemic side-effects, such as skin thickening, Cushing' syndrome and glycosuria.

Tretinoin is also sometime prescribed, however, no validated results demonstrated that SMD actually improve with the topical application of tretinoin.

Therefore, the local treatments with antihistamine antibiotics and corticosteroids, are prophylaxis that cannot relief the pro-inflammatory hemosiderin depots in SMD.

It is therefore evident that the present dermatological treatments do not provide satisfactory recovery from SMD.

On the other hand, we have first observed that hydroxypyridonones were so far conceived for the cure of systemic diseases including atheroschlerosis (WO02254650), HIV infections (WO022465; WO9955676), in thrombin inhibition (WO0179262, WO9730708), in sexual disfunction (WO0007595 and in transfusion-dependent iron overload subjects (USRE35,948; PCT/FR97/01211; WO0202114).

WO 98/130 43 discloses the use of 1-hydroxy-2 pyridones for the treatment of skin diseases caused by bacteria and fungi and acne rosazea.

In our previous patent application (WO01/17486) hydroxypyridonones we applied onto hyperpigmented skin, i.e. in areas of a dark colour caused by excess of melanin production with or without the concomitant occurrence of hemosiderin deposits.

However, a further clinical experience indicated that the inhibitory activity of 1,2-dialkyl-3-hydroxy-pyridonones is insufficient to cope with the request of hypermelanosis. Conversely, a good melanin inhibition was accomplished by the 1,2-unsubstituted 3-hydroxypyridonones. However, the latter exert toxic and irritant effects on skin, which in turns discouraged us to pursue their development as whitening agents.

Instead, an effective and safe treatment of the pro-inflammatory condition and the relief of the hemosiderin burdens in subjects affected by SMD like rosacea, capillaritis and other skin microcirculatory disorders was still desired.

### DESCRIPTION OF THE INVENTION

We have now discovered that the application of hydroxypyridonone in effective amounts as external agent for patients suffering from skin microcirculatory disorders (SMD) provides for a significant amelioration of sufferers conditions.

Furthermore, we have found out that also capillaritis-related disorders from hemorrhagic events and capillary-venular degenerative patterns, such as traumatic lesions, drug-induced and actinic purpura may be also treated with said hydroxypyridonones.

According our findings, the local application of hydroxypyridonones provide a anti-inflammatory action combined with the depletion of hemosiderin, thus offering a better treatment of SMD compared to corticosteroids or anti-histaminics.

Accordingly, one object of the present invention is a new use of hydroxypyridonones for the preparation of a topical medicament for treating SMD, said medicament comprising at last a compound of formula (I): 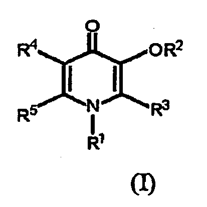 wherein:
R¹ represents a (C₁-C₁₀)-alkyl, (C₁-C₁₀)-alkenyl, (C₁-C₁₀)-alkoxy, (C₁-C₁₀)-hydroxyalkyl, (C₅-C₁₂)-aralkyl, (C₃-C₁₂)-cycloalkyl, (C₁-C₈)-carboalkoxy or (C₁-C₈)-carbamyl, or a (C₁₀-C₃₀)-peptide or peptidomimetic moiety, or a (C₃-C₆)-polyol or monosaccharide;
R² represents an hydrogen atom or a linear or branched, saturated or unsaturated (C₁-C₂₂)-acyl, optionally substituted by (C₁-C₈)-alkoxy, carboxy, (C₁-C₈) alkoxycarbonyl, amino, hydroxy, said amino and hydroxy being optionally (C₁-C₂₂)-acylated or -alkylated;
R³, R⁴ and R⁵, each individually, represent a hydrogen atom, or (C₁-C₁₀)-alkyl, (C₁-C₁₀)-alkenyl, (C₁-C₁₀)-alkoxy, (C₅-C₁₂ aryl) alkyl, (C₅-C₁₂)-cycloalk_{Y}l, (C₁-C₈ carbo)-alkoxy or (C₁-C₈)-carbamyl group; with the proviso that both R¹ and R³ are not hydrogen;
and dermatologically/cosmetically salts thereof.

Also included are novel 3-hydroxy-4-pyridinones derivatives from aminomonosaccharides or aminoitols, such as those disclosed in U.S. Pat. No. 6,177,409.

Compounds suitable for our purpose are hydroxypyridonones of formula (I), particularly preferred are those wherein R², R³, R⁴, and R⁵ are hydrogens, while R¹ and R³ are (C₁-C₄)-alkyl, hydroxyalkyl or -alkoxy groups.

More particularly, preferred compound suitable for our purpose include 1,2-dimethyl-3-hydroxy-4-pyridinone (deferiprone); 1,2-diethyl-3-hydroxy-4-pyridinone; 1-methyl-2-ethyl-3-hydroxy-4-pyridinone; 1-methyl-2-ethyl-3-hydroxy-4-pyridinone; 1-methyl-2-(2-methoxy-ethyl)-3-hydroxy-4-pyridinone.

Other preferred compound are the acyl esters, wherein R² is an acyl group acting as pro-drug and the 3-hydroxy group is then reconverted thereto in vivo.

Exemplary acyl group are those formed by unbranched, naturally occurring fatty acids. The naturally occurring fatty acids embraced by the invention include C8:0 (caprylic acid), C10:0 (capric acid), C12:0 (lauric acid), C14:0 (myristic acid), C16:0 (palmitic acid), C16:1 (palmitoleic acid), C16:2, C18:0 (stearic acid), C18:1 (oleic acid), C18:1-7 (vaccenic), C18:2-6 (linoleic acid), C18:3-3 (alpha-linolenic acid), C18:3-5 (eleostearic), C18:3-6 (delta-linolenic acid), C18:4-3, C20:1 (gondoic acid), C20:2-6, C20:3-6 (dihomo-y-linolenic acid), C20:4-3, C20:4-6 (arachidonic acid), C20:5-3 (eicosapentaenoic acid), C22:1 (docosenoic acid), C22:4-6 (docosatetraenoic acid), C22:5-6 (docosapentaenoic acid), C22:5-3 (docosapentaenoic), C22:6-3 (docosahexaenoic acid) and C24:1-9 (nervonic). Highly preferred naturally occurring fatty acids with 14 to 22 carbon chains and mixture thereof.

Alternatively, the acyl group may be a C₁₋₈ acyl, especially a C₈₃₋₇ acyl branched at the carbon atom adjacent to the carbonyl group, such as an isopropyl or t-butyl group.

The compounds may be used in the form of a dermatologically/cosmetically acceptable salt. These salts may be of two types, being formed with either dermatologically/cosmetically acceptable bases or acids. Thus salts may be formed between the anion produced by the loss of the 3-hydroxy group proton and a base derived cation. Examples of suitable bases are the alkali metal hydroxides, for example sodium hydroxide, quaternary ammonium hydroxides and amines such as tris (tris representing 2-amino-2-hydroxymethyl propane 1,3-diol). More preferably, salts may be formed through protonation of the carbonyl function at the 4-position of the 3-hydroxypyridinone ring by an acid. Suitable acids may be inorganic or organic. Examples of such inorganic acids are phosphoric acid, nitric acid, sulphuric acid and particularly an hydrohalic acids, e.g. hydrochloric acid. Examples of such organic acids are citric acid, oxalic acid, fumaric acid, maleic acid, lactic acid, succinic acid, malic acid, tartaric acid and methane sulphonic acid.

The aforementioned compounds of formula (I) decrease the inflammatory pattern when applied to a skin affected by SMD and deplete hemosiderin stores in skin.

The hydroxypyridonones deferiprone is an iron chelator that has a partition coefficient 21 times that of deferoxamine. Recent work shows that the cellular uptake of deferiprone is significantly higher than that of deferoxamine. (Berdoukas et al, Lancet 341:1088, 1993). Other studies have shown the deferiprone's ability to dramatically decrease lipid peroxidation, hydroxyl radical production, and free radical-mediated cell damage (Kamiyama et al, Neurol Med Chir 21:201-209, 1981).

Although not intending to be bound by theory, there are evidences to support the hypothesis that the protective effect of deferiprone results from the iron-binding capacity of the compound and from the prevention of free radical production. The deferiprone's ability to dramatically decrease lipid peroxidation, hydroxyl radical production, and free radical-mediated cell damage has already been established (Morel et al, Free Radic Biol Med 13:499-508, 1992). Substituted hydroxypyridonones such as deferiprone and analogues thereof are attractive candidates for dermatology therapy because of good dermal absorption and low incidence of serious side effects. The present findings provide a clear indication that hydroxypyridonones can be of benefit in the treatment of SMD.

The hydroxypyridonones, unlike other chelators such as deferoxamine, has been shown to rapidly penetrate and chelate hemosiderin stores in skin.

According to an aspect of the invention we have provided a new method of use of compounds of formulae (I-III) for the preparation of a topical medicament for decreasing the inflammatory patters in a patient suffering from SMD.

According to another aspect of the invention we have provided a new use of compounds of formula (I) for the preparation of a topical medicament for depleting the cutaneous hemosiderin deposits in a patient suffering from SMD.

Therefore, according to these particular embodiments, compound of formula (I) are most preferably applied in the form of appropriate "topical medicament".

The expression "topical medicament" as used herein, means a composition which may deliver the compound of formula (I) by external and intradermal routes on skin affected by microcirculatory skin disorders (SMD).

Exemplary non-limiting SMD include rosacea, cutaneous vasculitis, contact allergy skin capillaritis, lichens aureus and actinic purpura.

Another exemplary non-limiting SMD is skin capillaritis, a term which groups pathology with common clinical features, also named by the dermatologist who first described them, namely: progressive pigmentary dermatosis (Schamberg's disease), purpura annularis telangiectodes (Majocchi disease), lichen aureus, itching purpura, eczematid-like purpura (Ducas-Kapetanakis purpura), and pigmented purpuric lichenoid dermatosis (Gougerot-Blum dermatosis).

Further exemplary non-limiting MSD include the traumatic intradermal haemorrhage, such as the complication of schlerotherapy, lipoplasty and tattooing as well as the drug-induced pigmented purpuric dermatosis..

The compounds of formula (I) show high dermal compatibility and low irritation behavior when applied to human skin affected of skin capillaritis and related disorders by the applications of topical, transdermal and intradermal medicaments, which are effective in the fields of medicaments and cosmetics.

The content of compounds of formula (I) as the active principle in the "topical medicament" varies according to the carrier system and the degree of SMD damage.

In general, the proportion of compounds of formula (I) is in from 0.05 to 50% by weight, preferably from 0.1 to 10% by weight, more preferably from 0.5 to 5% by weight, in admixture with customary auxiliary agents.

Compounds of formula (I) are to a large extent known compounds, and can be synthesized according to general procedures, such those described by Kontoghiorghes & Sheppard in Inorg. Chim. Acta 136:L11-L12 (1987); Zhang et al., in Can. J. Chem. 70:763-770 (1992); Hider et al. in GB2118176; Bartulin et al., in J. Heterocyclic Chem. 29:1017-1019 (1992); or Ghisalberti in MI2002A 001560 (2001).

According to another aspect of the invention we have provided a treatment of SMD comprising the topical application to the patient in need thereof of a therapeutically effective amount of a hydroxypyridonone compound of formula (I ).

The compounds of formula (I) used in the method of the present invention are most preferably applied in the form of appropriate cosmetic or dermatological compositions which comprise dermatologically/cosmetically acceptable ingredients.

The expression "dermatologically/cosmetically acceptable ingredients" designate in the present specification products which are suitable for their use in topical treatments, for example those included in the INCI list issued in 96/335/EC "Annex to Commission Decision of 8 May 1996" and further modifications.

Suitable compositions contain the active ingredient and a skin-acceptable carrier. They may take a wide variety of forms such as, for example, solid forms, e.g. powders; liquid forms, e.g. solutions, gelled solutions or suspensions in aqueous or oily mediums; semi-liquid formulations, e.g. creams, gellies, pastes, ointments, and salves comprising as the active principle as well as suitable carriers.

In some cases the use may be made of covers, e.g. plasters, bandages, dressings, gauze pads and the like which have been impregnated or sprinkled with a liquid formulation containing the active agent, e.g. with an aseptic aqueous solution, or strewed with a solid composition, smeared, covered or coated with a semi-liquid composition.

According to another preferred embodiment, the invention concerns a method for the local treatment of SMD which comprises administering an effective amount of a compound of formula (I) by the intradermal route. More particularly, for such an intradermal route several tool can be applied, including iontophoresis or local injection, e.g. syringe or dermojet, in the latter modes of application compound of formula (I) will conveniently suspended in a sterilized liquid formulation.

More particularly, for such an intradermal technique of administration, the topical medicament suitable for our purpose may be applied by an external formulation which comprise a percutaneous penetration enhancer that augments delivery of active ingredient to the dermal layers. Percutaneous penetration enhancers include chemical and physical enhancers, and mixtures thereof.

Preferred chemical enhancers are dermatologically and cosmetically acceptable chemical enhancers, e.g. alpha-hydroxyacids, glycerine, and those discussed by Smith EW & Maibach HI, eds., in "Percutaneous penetration enhancers", CRC Press, NY, 1995.

Physical enhancers include those capable to enhance skin porosity and/or hydration, such as occlusion devices, hydrocolloid patches and other intradermal delivery systems, lipophilic penetrants including liposomes, microemulsions and lipospheres, delipidization, electroporation, ultrasound, iontophoresis, and the like methods.

According to another embodiment, the compound of formula (I) may also be applied by a "short-contact method" to treat SMD. The "short-contact method", as used herein, is intended to distinguish over conventional, or extended-contact, methods of treatment where the topical medicament is applied to a patient's skin and left indefinitely or until routine washing occurs after a prolonged period of time, typically overnight.

The short-contact method is intended to comprise the steps of applying a composition of invention to an affected area of the skin for a brief time period followed by rinsing of the skin area. For, the usual contact time is from about 30 seconds to about 30 minutes, preferably for a period of from about 5 to about 10 minutes. Immediately following the application, the skin is rinsed thoroughly, typically with lukewarm water.

Exemplary short-contact method is the application of a peeling gel comprising from 10% to 70% by weight of composition of an alpha-hydroxy acid, such as glycolic acid, lactic acid or mixture thereof, or of a beta-hydroxy acid such as salicylic acid.

The short-contact method is generally applied to the affected areas once or more times during the day, preferably at least twice a day, and repeated at least 3 times a week.

For the intended treatment, the compositions of the invention are administered 2 to 4 times per day, preferably 1 to 3 times, advantageously at least 2 times per day. The overall duration of the treatment is continued for as long as the conditions exist, i.e., until the SMD are relieved, as can readily be determined by the patient's doctor.

Other synergistic ingredients can be used in combination with compound of formula (I). Although not limited to this categories, general examples include UV-absorbers, antioxidants, anti-wrinkles, anti-inflammatory agents (steroidal and NSAIDS), antibiotics, antifungals, vitamins, anti-acne agents, anti-histaminics, depigmentors, etc.

Preferred synergistic ingredients also include chelators such as catechols (e.g. *N,N-*dimethyl-2,3-dihydroxybenzamide, enterobactin, MECAM, (Et)₃MECAM, TRENCAM), hydroxamates (e.g. acetohydroxamic acid, desferrioxamine (DFO)), aminocarboxylates (e.g. diethyl triaminopentaacetic acid (DTPA), *N,N*'-bis(2-hydroxybenzyl)-ethylenediamine-*N*,*N*'-diacetic acid (HBED), ethylenediaminetetraacetic acid (EDTA), diethylenetriaminepentaacetic acid (DTPA)), hydroxycarboxylates (citratic acid, tartaric acid, staphloferrin, rhizoferrin), desferrithiocins (e.g. desferrithiocin (DFT)), triazoles (e.g. ICL670A-Novartis), PIH analogs (pyridoxal isonicotinoyl hydrazone (PIH), salicylaldehyde isonicotinoyl hydrazone (SIH)) and condensed pyridines (e.g. 2,2'-bipyridyl, 1,10-phenanthroline).

Further preferred synergistic ingredients include endothelial reinforcing agents, such as triterpenoids of Centella asiatica (e.g. asiatic acid, madecassic acid, asiaticoside, madecaside), Ginseng metabolites (e.g. giberellic acid, gibberellin hormone-like molecules, ruscogenin, escin, esculin, troxerutin, anthocyanes, proanthocyanosides, and phytic acid.

Further particularly preferred synergistic ingredients include angiogenic agents such as prostaglandin (e.g. PGE1,2), lipid amides (e.g. erucamide), lipid metabolites (e.g. 12(R)-hydroxyeicosatrienoic acid), saponins (e.g. Ginseng radix rubra saponins), phytosterols (e.g. β-sitoserol form Aloe vera gel), plant polyphenols (e.g. cinnamic and benzoic acids derivatives form Populus nigra), salvianolides (from Salvia miltiorrhiza, salvianolic acid B). adenosine, inosine, hypoxanthine, nicotine, and nicotinamide.

The following examples are intended to illustrate the scope of the present invention.

### EXAMPLES

### Examples 1-38 - Applicative models

Hydroxypyridonones are a family of compounds exhibiting have a broad range of physico-chemical features. A key feature is the hydro-lipophilic property, which basically correlate with its distribution coefficients ("Kow"). Following the determination of Kow = distribution coefficient, calculated as partition between water and 1-octanol of the hydroxypyridinones-HCl addition salt, according Dubbin et al. (J Med Chem, 1993, 36, 2448-58) the following delivery systems are preferably applied: gel for hydroxypyridonones with Kow ≥ 1 (for example glycolic gel); alcoholic lotion for Kow between 1 and 10; and emulsion or ointment for Kow ≥ 10, as shown in Table I.

**TABLE I - Delivery system for 3-hydroxy-4-pyridonones of formula (I)**

| Ex N° system | R¹ | R² | R³ | R⁴ | R⁵ | Kow | Delivery |
|---|---|---|---|---|---|---|---|
| 1 | -CH₃ | H | -CH₃ | H | H | 0.17 | glycolic gel |
| 2 | -CH₂CH₃ | H | -CH₃ | H | H | 0.49 | glycolic gel |
| 3 | (CH₂)₂CH₃ | H | -CH₃ | H | H | 1.51 | alcoholic lotion |
| 4 | CH(CH₂)₂ | H | -CH₃ | H | H | 1.12 | alcoholic lotion |
| 5 | -(CH₂)₃CH₃ | H | -CH₃ | H | H | 5.05 | alcoholic lotion |
| 6 | -(CH₂)₄CH₃ | H | -CH₃ | H | H | 17.4 | O/W emulsion |
| 7 | -(CH₂)₅CH₃ | H | -CH₃ | H | H | 79.5 | O/W emulsion |
| 8 | -(CH₂)₇CH₃ | H | -CH₃ | H | H | 750 | O/W emulsion |
| 9 | -(CH₂)₉CH₃ | H | -CH₃ | H | H | n.a. | O/W emulsion |
| 10 | -CH₂CH=CH₂ | H | -CH₃ | H | H | 8.30 | O/W emulsion |
| 11 | -CH₃ | H | -CH₂CH₃ | H | H | 0.63 | alcoholic lotion |
| 12 | -CH₂CH₃ | H | -CH₂CH₃ | H | H | 1.78 | alcoholic lotion |
| 13 | -(CH₂)₂CH₃ | H | -CH₂CH₃ | H | H | 5.04 | alcoholic lotion |
| 14 | -CH(CH₂)₂ | H | -CH₂CH₃ | H | H | 5.40 | alcoholic lotion |
| 15 | -(CH₂)₃CH₃ | H | -CH₂CH₃ | H | H | 16.6 | O/W emulsion |
| 16 | -(CH₂)₅CH₃ | H | -CH₂CH₃ | H | H | 189 | O/W emulsion |
| 17 | -CH₃ | oleoyl | -CH₃ | H | H | > 200 | O/W emulsion |
| 18 | -CH₃ | palmitoyl | -CH₃ | H | H | > 200 | O/W emulsion |
| 19 | -CH₃ | stearoyl | -CH₃ | H | H | > 200 | O/W emulsion |
| 20 | -(CH₂)₂OH | H | -CH₃ | H | H | 0.08 | glycolic gel |
| 21 | -(CH₂)₂OH | H | -CH₂CH₃ | H | H | 0.22 | glycolic gel |
| 22 | -(CH₂)₃OH | H | -CH₃ | H | H | 0.13 | glycolic gel |
| 23 | -(CH₂)₄OH | H | -CH₃ | H | H | 0.18 | glycolic gel |
| 24 | -(CH₂)₂COOH | H | -CH₃ | H | H | <0.001 | glycolic gel |
| 25 | -(CH₂)₃COOH | H | -CH₃ | H | H | <0.001 | glycolic gel |
| 26 | -(CH₂)₂CONHMe | H | -CH₃ | H | H | 0.08 | glycolic gel |
| 27 | -(CH₂)₂CONHEt | H | -CH₃ | H | H | 0.15 | glycolic gel |
| 28 | -(CH₂)₂CONHPr | H | -CH₃ | H | H | 0.40 | glycolic gel |
| 29 | -(CH₂)₂CONHBu | H | -CH₃ | H | H | 1.25 | alcoholic lotion |
| 30 | -(CH₂)₂CON(Me)₂ | H | -CH₃ | H | H | 0.07 | glycolic gel |
| 31 | -(CH₂)₂CON(Et)₂ | H | -CH₃ | H | H | 0.44 | glycolic gel |
| 32 | -(CH₂)₂NH₂ | H | -CH₃ | H | H | 0.03 | glycolic gel |
| 33 | -(CH₂)₃NH₂ | H | -CH₃ | H | H | 0.01 | glycolic gel |
| 34 | -(CH₂)₂OCH₃ | H | -CH₃ | H | H | 0.39 | glycolic gel |
| 35 | -(CH₂)₂OCH₃ | H | -CH₂CH₃ | H | H | 1.1 | alcoholic lotion |
| 36 | -(CH₂)₂OCH₂CH₃ | H | -CH₃ | H | H | 1.32 | alcoholic lotion |
| 37 | -(CH₂)₃OCH₂CH₃ | H | -CH₂CH₃ | H | H | 5.2 | alcoholic lotion |
| 38 | -CH(CH₃)CH₂OCH₃ | H | -CH₃ | H | H | 0.55 | glycolic gel |

| | | | | | | | |
|---|---|---|---|---|---|---|---|
| Formulative Examples A, B and C - Delivery system models (figure as g per 100 g) | | | | | | | |

Typical model carriers designed taking into account the Kow of the active ingredients are illustrated in these Examples.

| emulsion | Example A Glycolic gel | Example B Alcoholic lotion | Example C O/W |
|---|---|---|---|
| compound of formula (I) | 1 - 4 (**) | 1- 4 (**) | 1-4 |
| sodium lauryl sulphate | 0 - 0.3 | - | 0-1 |
| polysorbate 80 (*) | 0-1 | - | 0-1 |
| octyl palmitate | - | - | 3-5 |
| myristyl myristate | - | 2-4 | 5-8 |
| glyceryl monostearate | - | 0.5 - 2 | 3-5 |
| cetearyl alcohol and | | | |
| ceteareth-20 | - | 2-4 | 5-8 |
| glycolic acid | 10-50 | - | 0- 10 |
| | | | |
| glycerine | 0-10 | - | 5-10 |
| disodium-EDTA | 0-0.1 | 0-0.1 | 0-0.1 |
| ethyl alcohol 95° v/v | 0-10 | 25 - 75 | 0-10 |
| xanthan gum | 0.5-1.5 | - | 0-0.5 |
| ammonia and HCl | qb to pH 2 to 4 | - | qb to pH 4 |
| to 7 | | | |
| preservatives | - | - | 0.2 - 0.5 |
| water | qb to 100 g | qb to 100 g | qb to 100 g |

| | | | |
|---|---|---|---|
| (*) polyoxyethylene-(20)-sorbitan monooleate (**) as HCl addition salt | | | |

Noteworthy, for high hydrophilic hydroxypyridonones the emulsifiers may be unnecessary in the glycolic gel. The hydroalcoholic vehicle is also preferred according to the solubility of the hydroxypyridonone. The high lipophilic hydroxypyridonones are incorporated in the oily phase of W/O emulsion, which is then emulsified at the melting temperature with the aqueous phase.

Capillaritis inflammation and deposition may be located at different level on cutis and can followed by monitoring hemosiderin depots in tissues and macrophages, CD4+ lymphocytes, CD1a+ dendritic cells, plasma cells and neutrophils. These infiltrates may have different proportions in individual and/or according to type of SMD being treated.

### Case History #1

A 63 year old subject with extensive progressive pigmented purpura involving the face and neck was treated utilizing a cream made essentially in accordance with Example A. The patient applied the gel regularly twice-a-day onto the affected area. The itching and inflammatory condition were relieved within one month of treatment, while most of the face nearly clear of purpura disorders and lesions, with the checks (the most intensely affected area) exhibited some residual trace of purpura.

### Case History #2

A 42 year old female subject with a series of tiny skin microhaemorrhages on both legs caused by a self-practice of hair-removal follow by hair re-growth beneath the skin was treated utilizing a cream made essentially in accordance with Example C. The patient applied the cream regularly once-a-day to the spots. Within one week, the itching and pruritus have been totally relieved, and the patient was free of the disorder.

### Case History #3

A 22 year old female subject with Schamberg's disease on both legs was successfully treated with a treatment similar to Case history #1.

### Case History #4

A 57 year old male subject with facial rosacea was successfully treated with a treatment similar to Case history #1.

### Case History #5

A 66 year old male subject with actinic purpura was successfully treated with a treatment similar to Case history #1.

### Case History #6

A 51 year old male subject with lichen aureus was successfully treated with a treatment similar to Case history #1.

## Claims

1. The use of a hydroxypyridinonc of formula (I): 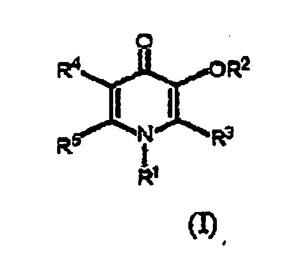
R¹ represents a (C₁-C₁₀)-alkyl, (C₁-C₁₀)-alkenyl, (C₁-C₁₀)-alkoxy, (C₁-C₁₀)-hydroxyalkyl, (C₅-C₁₂)-aralkyl, (C₃-C₁₂)-cycloalkyl, (C₁-C₈)-carboalkoxy or (C₁-C₈)-carbamyl, or a (C₃-C₆)-polyol or monosaccharide;
R² represents an hydrogen atom or a linear or branched, saturated or unsaturated (C₁-C₂₂)-acyl, optionally substituted by (C₁-C₈)-alkoxy, carboy, (C₁-C₈)-alkoxycarbonyl, amino, hydroxy, said amino and hydroxy being optionally (C₁-C₂₂)-acylated or -alkylated;
R³, R⁴ and R⁵, each individually, represent a hydrogen atom, or (C₁-C₁₀)-alkyl, (C₁-C₁₀)-alkenyl, (C₁-C₁₀)-alkoxy, (C₅-C₁₂ aryl) alkyl, (C₅-C₁₂)-cycloalkyl, (C₁-C₈ carbo)-alkoxy or (C₁-C₈)-carbamyl group; with the proviso that both R¹ and R³ are not hydrogen;
and dermatologically/cosmetically salts thereof,
for the manufacture of a topical medicament useful in the treatment of a skin microcirculatory disorder (SMD) selected from rosacea, cutaneous vasculitis, actinic purpura and skin capillaritis.

2. Use according to claim 1, wherein the skin capillaritis is selected in the group consisting of purpura annularis telangiectodes, lichen aureus, contact allergy skin capillaritis, itching purpura and eczematid-like purpura.

3. Use according to claim 1, wherein R¹ and R² are methyl, R³ and R⁴ are hydrogens.

4. Use according to claim 1, wherein R¹ and R² are ethyl R³ and R⁴ are hydrogens.

5. Use according to claim 1, wherein R¹ is CH₂CH₂OH, R² is methyl or ethyl, and R³ and R⁴ are hydrogens.

6. Use according to claim 1, wherein the hydroxypyridinone of formula (I) is selected among 1,2-dimethyl-3-hydroxy-4-pyridinone; 1,2-diethyl-3-hydroxy-4-pyridinone and 1-methyl-2-ethyl-3-hydroxy-4-pyridinone.

## Patentansprüche

1. Die Verwendung eines Hydroxypyridinons der Formel (I): 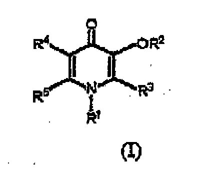 wobei R¹ ein (C₁-C₁₀)-Alkyl, (C₁-C₁₀)-Alkenyl, (C₁-C₁₀)-Alkoxy, (C₁-C₁₀)-Hydroxyalkyl, (C₅-C₁₂)-Aralkyl, (C₃-C₁₂)-Cycloalkyl, (C₁-C₈)-Carboalkoxy oder (C₁-C₈)-Carbamyl, oder ein (C₃-C₆)-Polyol oder Monosaccharid darstellt;
R² ein Wasserstoffatom oder ein lineares oder verzweigtes, gesättigtes oder ungesättigtes (C₁-C₂₂)-Acyl, optional substituiert mit (C₁-C₈)-Alkoxy, Carboxy, (C₁-C₈)-Alkoxycarbonyl, Amino, Hydroxy darstellt, wobei besagtes Amino oder Hydroxy optional (C₁-C₂₂)-acyliert oder alkyliert ist;
R³, R⁴ und R⁵ jeweils individuell, ein Wasserstoffatom oder eine (C₁-C₁₀)-Alkyl-, (C₁-C₁₀)-Alkenyl-, (C₁-C₁₀)-Alkoxy-, (C₅-C₁₂ Aryl)alkyl-, (C₅-C₁₂)-Cycloalkyl-, (C₁-C₈ Carbo)alkoxy- oder (C₁-C₈)-Carbamylgruppe darstellen; unter der Voraussetzung, dass R¹ und R³ nicht beide Wasserstoff sind;
und dermatologisch/kosmetische Salze hiervon,
zur Herstellung eines topischen Medikamentes zur Behandlung von Störungen der Mikrozirkulation der Haut (SMD) ausgewählt aus Rosacea, kutaner Vaskulitis, aktinischer Purpura und Kapillaritis der Haut.

2. Die Verwendung gemäß Anspruch 1, wobei die Kapillaritis der Haut ausgewählt ist aus der Gruppe, bestehend aus Purpura Anularis Teleangiectodes, Lichen Aureus, Kontaktallergie-Kapillaritis der Haut, juckender Purpura und ekzematös-artiger Purpura.

3. Die Verwendung gemäß Anspruch 1, wobei R¹ und R² Methyl sind, R³ und R⁴ Wasserstoffe sind.

4. Die Verwendung gemäß Anspruch 1, wobei R¹ und R² Ethyl sind, R³ und R⁴ Wasserstoffe sind.

5. Die Verwendung gemäß Anspruch 1, wobei R¹ CH₂CH₂OH ist, R² Methyl oder Ethyl ist und R³ und R⁴ Wasserstoffe sind.

6. Die Verwendung gemäß Anspruch 1, wobei das Hydroxypyridinon der Formel (I) ausgewählt ist aus 1,2-Dimethyl-3-hydroxy-4-pyridinon, 1,2-Diethyl-3-hydroxy-4-pyridinon und 1-Methyl-2-ethyl-3-hydroxy-4-pyridinon.

## Revendications

1. Utilisation d'une hydroxypyridinone de formule (I) : 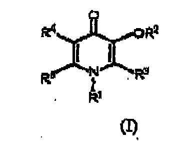
R¹ représente un alkyle en (C₁-C₁₀), un alcényle en (C₁-C₁₀) , un alcoxy en (C₁-C₁₀), un hydroxyalkyle en (C₁-C₁₀), un aralkyle en (C₅-C₁₂), un cycloalkyle en (C₃-C₁₂), un carboalcoxy en (C₁-C₈), ou un carbamyle en (C₁-C₈) ou un polyol en (C₃-C₆) ou un monosaccharide,
R² représente un atome d'hydrogène ou un acyle en (C₁-C₂₂), éventuellement substitué par un alcoxy en (C₁-C₈), un carboxy, un alcoxycarbonyle en (C₁-C₈), un amino, un hydroxy, ledit amino et hydroxy étant éventuellement acylé ou alkylé en (C₁-C₂₂) ;
R³, R⁴ et R⁵, chacun individuellement, représentent un atome d'hydrogène, ou un alkyle en (C₁-C₁₀), un alcényle en (C₁-C₁₀), un alcoxy en (C₁-C₁₀), un alkyle (aryle en C₅-C₁₂), un cycloalkyle en (C₅-C₁₂), un carboalcoxy en (C₁-C₈) ou un groupe carbamyle en (C₁-C₈), à condition que R¹ et R³ ne soient pas un hydrogène ;
et leurs sels dermatologigues/cosmétiques,
pour la fabrication d'un médicament topique utile dans le traitement d'un trouble micro-circulatoire de la peau (SMD) choisi parmi l'acné rosacée, la vasculite cutanée, le purpura actinique, et la capillarite dermique.

2. Utilisation selon la revendication 1, dans laquelle à capillarite dermique est choisie dans le groupe constitué de purpura annulaire télangiectasique, de lichen aureus, de capillarite dermique d'allergie par contact, le purpura urticant et le purpura semblable à l'eczématide.

3. Utilisation selon la revendication 1, dans laquelle R¹ et R² sont un méthyle, R³ et R⁴ sont des hydrogènes.

4. Utilisation selon la revendication 1, dans laquelle R¹ et R² sont un éthyle, R³ et R⁴ sont des hydrogènes.

5. Utilisation selon la revendication 1, dans laquelle R¹ est un CH₂CH₂OH, R² est un méthyle ou éthyle, et R³ et R⁴ sont des hydrogènes.

6. Utilisation selon la revendication 1, dans laquelle l'hydroxypyridinone de formule (I) est choisie parmi une 1,2-diméthyl-3-hydroxy-4-pyridinone ; 1,2-diéthyl-3-hydroxy-4-pyridinone et 1-méthyl-2-éthyl-3-hydroxy-4-pyridinone.
